# EUROPEAN PATENT SPECIFICATION

(11) **EP 3 295 174 B1**
(45) Date of publication and mention of the grant of the patent: **04.11.2020**
(21) Application number: 16793373.8
(22) Date of filing: 10.05.2016
(51) Int. Cl.: G01N 33/53, G01N 33/533, G01N 33/543, G01N 33/58, G01N 33/536

(54) **METHOD FOR RE-USING TEST PROBE AND REAGENTS IN AN IMMUNOASSAY**
VERFAHREN ZUR WIEDERVERWENDUNG EINER PRÜFSONDE UND VON REAGENZIEN IN EINEM IMMUNOASSAY
PROCÉDÉ DE RÉUTILISATION D'UNE SONDE D'ESSAI ET DE RÉACTIFS DANS UN IMMUNODOSAGE

(30) Priority: 11.05.2015 US 201562159919 P
(43) Date of publication of application: 21.03.2018
(73) Proprietor: Access Medical Systems, Ltd., Palo Alto, CA 94303 (US)
(72) Inventor: ZUK, Robert F., Palo Alto, California 94304 (US); XIA, Qing, Palo Alto, California 94304 (US)
(74) Representative: Crease, Devanand John
(86) International application number: PCT/US2016/031661
(87) International publication number: WO 2016/183092

(56) References cited:
- WO-A1-2013/158494
- WO-A2-2010/101931
- US-A1- 2009 023 144
- US-A1- 2013 052 081
- US-A1- 2013 052 081
- US-A1- 2013 295 591
- US-B2- 8 647 889
- US-B2- 8 753 574
- WIJESURIYA D ET AL: "Regeneration of immobilized antibodies on fiber optic probes", BIOSENSORS AND BIOELECTRONICS, ELSEVIER SCIENCE LTD. UK, AMSTERDAM, NL, vol. 9, no. 8, 1 January 1994 (1994-01-01) , pages 585-592, XP026604245, ISSN: 0956-5663, DOI: 10.1016/0956-5663(94)80051-0 [retrieved on 1994-01-01]
- YUHI T. ET AL.: 'Gold nanoparticle based immunochromatography using a resin modified micropipette tip for rapid and simple detection of human chorionic gonadotropin hormone and prostate-specific antigen.' SCIENCE AND TECHNOLOGY OF ADVANCED MATERIALS vol. 7, no. 3, 2006, pages 276 - 281, XP028057384

## Description

### BACKGROUND OF THE INVENTION

Cost containment is a major goal for healthcare providers worldwide. In vitro diagnostics (IVD) is no exception, where the clinical utility of biomarkers in the diagnosis and prognosis has become standard in patient management. Immunoassay technology is large portion of the IVD industry and is steadily growing, about 3%/year in the U.S. and 15-20%/year in developing countries. In some cases, such as serial measurements for cardiac markers in diagnosing myocardial infarction, cost can limit the appropriate amount of testing.

Typical approaches to reducing the cost of immunoassays entail minimizing manufacturing expenses for materials, labor, and facilities overhead.

There is a need for reducing the cost of immunoassays, while maintaining the clinical performance at the same time.

### BRIEF DESCRIPTION OF THE DRAWINGS

FIG.1 illustrates an embodiment of the fluorescent detection system.
FIG. 2 illustrates one embodiment of the assay format, in which C-reactive protein (CRP) is an example of an analyte to be measured. The solid phase (probe tip) is immobilized with streptavidin:biotin-anti-CRP antibody.
FIG.3 illustrates an assay protocol of transferring probe.
FIG.4 illustrates an assay protocol of transferring probe in two sequences of events in a wide-range protocol.
FIG.5 illustrates the fluorescent signals of CRP samples at 30, 100, and 300 mg/L over 20 cycles of measurements (Sequence 1) by re-using the same test tube, with C30 antibody as a capture antibody and C5 antibody as a signal antibody.
FIG.6 illustrates the fluorescent signals of CRP samples at 10, 30, 100, and 300 mg/L over 9 cycles of measurements (Sequence 1) by re-using the same test tube, with C30 antibody as a capture antibody and C5 antibody as a signal antibody.
FIG. 7 illustrates the fluorescent signals of CRP samples at 0, 3, and 10 mg/L over 9 cycles of measurements (Sequence 2) by re-using the same test tube, with C30 antibody as a capture antibody and C5 antibody as a signal antibody.
FIG. 8 illustrates the reproducibility of fluorescent signals of CRP high to low samples at 3 and 300 mg/L, with C30 antibody as a capture antibody and C5 antibody as a signal antibody.
FIG. 9 show the correlation of the CRP results of 100 clinical samples measured by the present protocol and by an established clinical instrument, the Siemens BN II.
FIG. 10 illustrates the fluorescent signals of CRP samples at 0, 3, 10, and 30 mg/L over 9 cycles of measurements, with C2 antibody as a capture antibody and C5 antibody as a signal antibody.
FIG. 11 illustrates the fluorescent signals of PCT samples at 0, 1, 2, 5, and 10 ng/mL over 5 cycles of measurements, with a polyclonal antibody as a capture antibody and C16B5 monoclonal antibody as a signal antibody.

### DETAILED DESCRIPTION OF THE INVENTION

### Definitions

Terms used in the claims and specification are to be construed in accordance with their usual meaning as understood by one skilled in the art except and as defined as set forth below.

"About," as used herein, refers to within ± 15% or within ± 10% of the recited value.

An "analyte-binding" molecule, as used herein, refers to any molecule capable of participating in a specific binding reaction with an analyte molecule. Examples include but are not limited to, (i) antigen molecules, for use in detecting the presence of antibodies specific against that antigen; (ii) antibody molecules, for use in detecting the presence of antigens; (iii) protein molecules, for use in detecting the presence of a binding partner for that protein; (iv) ligands, for use in detecting the presence of a binding partner; or (v) single stranded nucleic acid molecules, for detecting the presence of nucleic acid binding molecules.

An "aspect ratio" of a shape refers to the ratio of its longer dimension to its shorter dimension.

A "binding molecular," refers to a molecule that is capable to bind another molecule of interest.

"A binding pair," as used herein, refers to two molecules that are attracted to each other and specifically bind to each other. Examples of binding pairs include, but not limited to, an antigen and an antibody against the antigen, a ligand and its receptor, complementary strands of nucleic acids, biotin and avidin, biotin and streptavidin, lectin and carbohydrates. Preferred binding pairs are biotin and streptavidin, biotin and avidin, fluorescein and anti-fluorescein, digioxigenin/anti-digioxigenin. Biotin and avidin, including biotin derivatives and avidin derivatives such as streptavidin, may be used as intermediate binding substances in assay protocols employing complex binding sequences. For example, antibodies may be labeled with biotin ("biotinylated") and used to bind to a target substance previously immobilized on a solid phase surface. Fluorescent compositions according to the present invention employing an avidin or streptavidin may then be used to introduce the fluorescent label.

"Immobilized," as used herein, refers to reagents being fixed to a solid surface. When a reagent is immobilized to a solid surface, it is either be non-covalently bound or covalently bound to the surface.

"A monolithic substrate," as used herein, refers to a single piece of a solid material such as glass, quartz, or plastic that has one refractive index.

A "probe," as used herein, refers to a substrate coated with a thin-film layer of analyte-binding molecules at the sensing side. A probe has a distal end and a proximal end. The proximal end (also refers to probe tip in the application) has a sensing surface coated with a thin layer of analyte-binding molecules.

A "wide range concentration", as used herein, refers to a concentration range over at least 50-fold, 100 fold, or 500-fold.

The present invention discloses a method to re-use an immunoassay test probe and reagents, from about 3 to 20 times, while maintaining acceptable clinical assay performance. The immunoassay test probe and reagents may be contained in one test strip, or one cartridge. The present invention re-uses test probe and reagents the and saves the cost on a per test basis.

There are several key elements to practice the invention. First, the invention regenerates the test probe by employing a denaturing reagent that disassociates the immune complexes bound to the antibodies immobilized on a solid phase, but does not denature or disassociate the antibodies bound to the solid phase to a degree that affects the assay performance. The denaturation step conditions the solid phase antibody for subsequent binding steps to other antigen containing samples. Second, the probe tip has a small dimension (< 5 mm in diameter) so that there is negligible consumption of the reagents, and no replenish of the reagents is necessary during the assay cycles. Third, the assay utilizes the same test probe and the same reagents necessary to perform a complete assay, which facilitates multiple assay cycles without additional reagents.

### Fluorescent Detection System

The present invention uses a fluorescent detection system as described in U.S. Patent No. 8,492,139 (also published as WO 2010/101931 A2), for measuring a fluorescent signal on a probe tip. Similar probes having a first antibody immobilized on the tip of the probe are described in WO 2013/158494 A1, US Patent No. 8,647,889 and US 2013/052081 A1. The system comprises: (a) a probe having an aspect ratio of length to width at least 5 to 1, the probe having a first end and a second end, the second end having a sensing surface bound with a fluorescent label; (b) a light source for emitting excitation light directly to the probe's sensing surface; (c) a collecting lens pointed toward the sensing surface; and (d) an optical detector for detecting the emission fluorescent light; where the collecting lens collects and directs the emission fluorescent light to the optical detector.

The probe can be a monolithic substrate or an optical fiber. The probe can be any shape such as rod, cylindrical, round, square, triangle, etc., with an aspect ratio of length to width of at least 5 to 1, preferably 10 to 1. Because the probe is dipped in a sample solution and one or more assay solutions during an immunoassay, it is desirable to have a long probe with an aspect ratio of at least 5 to 1 to enable the probe tip's immersion into the solutions. Heterogeneous assays can be performed where the long probe is transferred to different reaction chambers. Dispensing and aspirating reagents and sample during the assay are avoided. The sensing surface of the probe is coated with analyte-binding molecules and bound with fluorescent labels.

Any light source that can emit proper excitation light for the fluorescent label is suitable for the present invention. A prefer light source is a laser that can emit light with wavelengths suitable for fluorescent labels. For example, the laser center wavelength is preferred to be 649 nm for Cy5 fluorescent dye. A suitable optical detector for detecting emission light is a photomultiplier tube (PMT), a charge coupled device (CCD), or a photodiode.

The light source and the optical detector including the collecting lens are mounted on the same side of the probe tip surface (the sensing surface). If the sensing surface faces down, they are both mounted below the tip surface. If the sensing surface faces up, they are both mounted above the tip surface. They are closer to the sensing surface than the other end of the probe. The sensing surface is always within the numeric aperture of the collecting lens. The probe can be, but does not have to be centrally aligned with the collecting lens.

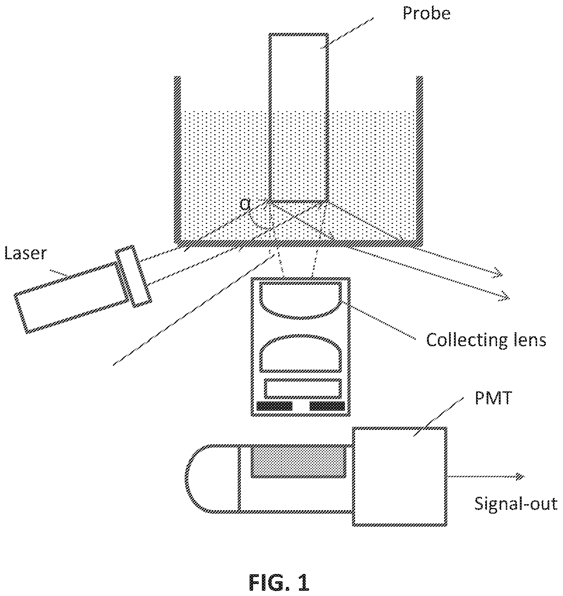

FIG. 1 illustrate an embodiment of the fluorescent detection system.

### Detecting An Analyte By A Recycling Protocol

The present invention is directed to a method of detecting an analyte in multiple liquid samples by a fluorescent immunoassay, using the same test probe and test reagents for different sample. The method comprises the steps of: (a) obtaining a probe having a first antibody immobilized on the tip of the probe, wherein the diameter of the tip surface is ≤ 5 mm; (b) dipping the probe in a pre-read vessel comprising an aqueous solution having pH of 6.0-8.5 to pre-read the fluorescent signal of the probe tip; (c) dipping the probe tip into a sample vessel containing a liquid sample having an analyte; (d) dipping the probe tip into a reagent vessel containing a reagent solution comprising a second antibody conjugated with one or more fluorescent labels to form an immunocomplex among the analyte, the first antibody, and the second antibody on the probe tip, wherein the first antibody and the second antibody are antibodies against the analyte; (e) dipping the probe tip into a washing vessel containing a wash solution; (f) determining the analyte concentration in the first sample by measuring the fluorescent signal of the immunocomplex at the probe tip, subtracting the pre-read fluorescent signal of (b), and quantitating against a calibration curve; (g) dipping the probe tip in an acidic solution having pH about 1.0-4.0 to elute the immunocomplex from the probe tip, and (h) repeat steps (b)-(f) 1-20 times, preferably 1-10 times, with a second liquid sample in a second sample vessel, whereby the analyte in multiple liquid samples is detected. The method uses the same probe, the same reagent solution, and the same washing solution in all cycles of reaction.

In step (a) of the present method, a probe that has a small tip for binding an analyte is obtained. The tip has a smaller surface area with a diameter ≤ 5 mm, preferably ≤ 2 mm or ≤ 1 mm. The small surface of the probe tip endows it with several advantages. In a solid phase immunoassays, having a small surface area is advantageous because it has less non-specific binding and thus produces a lower background signal. Further, the reagent or sample carry over on the probe tip is extremely small due to the small surface area of the tip. This feature makes the probe tip easy to wash, and causes negligible contamination in the wash solution since the wash solution has a larger volume. Another aspect of the small surface area of the probe tip is that it has small binding capacity. Consequently, when the probe tip is immersed in a reagent solution, the binding of the reagent does not consume a significant amount of the reagent. The reagent concentration is effectively unchanged. Negligible contamination of the wash solution and small consumption of the reagents enable the reagents and the wash solution to be re-used many times, for example, 3-20 times.

Methods to immobilize a first antibody to the solid phase (the sensing surface of the probe tip) are common in immunochemistry and involve formation of covalent, hydrophobic or electrostatic bonds between the solid phase and antibody. The first antibody, also called capture antibody for its ability to capture the analyte, can be directly immobilized on the sensing surface. For example, a first antibody can be first immobilized either by adsorption to the solid surface or by covalently binding to aminopropylsilane coated on the solid surface. Alternatively, the first antibody can be indirectly immobilized on the sensing surface through a binding pair. For example, the first antibody can be labeled with biotin by known techniques (see Wilchek and Bayer, (1988) Anal. Biochem. 171:1-32), and then be indirectly immobilized on the sensing surface coated with streptavidin. Biotin and streptavidin are a preferred binding pair due to their strong binding affinity, which does not dissociate during the low pH (pH 1-4) regeneration steps of the present method. The capture antibody immobilized on the sensing surface must be able to survive the denaturation condition when the probe sensing surface is regenerated to remove the immunocomplex bound to the sensing surface after the immunoreaction. The capture antibody immobilized on the sensing surface must not lose a significant amount of activity or significantly disassociate from the solid phase so that the immunoassay performance is compromised.

In step (b), the fluorescent signal of the probe tip is pre-read by a fluorescent detection system in a read vessel (or a read chamber, or a read well). The read vessel contains an aqueous solution such as water or a buffer having pH between 6.0 to 8.5. Preferably, the aqueous solution contains 1-10 mM or 1-100 mM of phosphate buffer, tris buffer, citrate buffer or other buffer suitable for pH between 6.0-8.5 , to neutralize the probe after low pH regeneration. Pre-read is necessary before the first sample binding to establish a baseline of any potential background fluorescence for the first cycle reaction. Pre-read is also necessary after the regeneration of the probe tip and before the next sample binding to establish a baseline for subsequent cycles. After each cycle, the pre-read signal can be the same, or higher, or lower than the pre-read signal of the previous cycle, due to the change of the binding property of the immobilized capture antibody caused by the denaturing condition. The inventor has discovered that for certain capture antibodies, the fluorescent signal at the completion of each cycle of reaction after subtracting the pre-read signal, remains constant for 20 cycles of reaction using the same probe and the same reagents. The inventor has also discovered that for other capture antibodies, the fluorescent signal continuously goes up or down slightly after each cycle of reaction, even after subtracting the pre-read signal. The acid treatment could alter the protein on the surface of the probe so that either the capture antibody binding capacity changes or the fluorescence signal is altered. Fluorescence is known to be very sensitive to environmental effects. In spite of the increase or decrease in fluorescence at each cycle, consistent quantification is obtained in such case with a cycle specific calibration; i.e., the fluorescent signal at the completion of each cycle of reaction, after subtracting the pre-read signal, is quantitated against a cycle-specific calibration curve included in the system.

In step (c) of the method, the probe tip is dipped into a sample vessel (or a sample chamber or a sample well), and incubated for 5 seconds to 5 minutes, 10 seconds to 2 minutes, or 30 seconds to 1 minute, to bind the analyte to the first antibody on the probe tip.

After step (c), the probe is optionally washed 1-5 times, preferably 1-3 times in a wash vessel (or a wash chamber or a wash well) containing a wash solution. This extra washing step may not be required because the amount of the carried-over solution is minimal due to a small binding surface area. The wash solution typically contains buffer and a surfactant such as Tween 20.

In step (d) of the method, the probe tip is dipped into a reagent vessel (or a reagent chamber or a reagent sell) for 5 seconds to 5 minutes, 10 seconds to 2 minutes, or 30 seconds to 1 minute, to bind the reagent to the analyte on the probe tip. The reagent solution comprises a fluorescent labelled second antibody (a signal antibody). Any suitable fluorescent label can be used in this method. An example of a fluorescent label is an arylsulfonate cyanine fluorescent dye as described in Mujumdar et al. (1993) Bioconjugate Chemistry, 4:105-111; Southwick et al. (1990) Cytometry, 11:418-430; and U.S. Pat. No. 5,268,486. Cy5 is a preferred arylsulfonate cyanine fluorescent dye, because it has a high extinction coefficient and good quantum yield; it also has fluorescent emission spectra in a range (500 nm to 750 nm) outside of the auto-fluorescence wavelengths of most biological materials and plastics. In addition, Cy5 has a good solubility in water, and has low non-specific binding characteristics.

A fluorescent label can covalently bind to a second antibody through a variety of moieties, including disulfide, hydroxyphenyl, amino, carboxyl, indole, or other functional groups, using conventional conjugation chemistry as described in the scientific and patent literature. Exemplary techniques for binding arylsulfonate cyanine fluorescent dye labels to antibodies and other proteins are described in U.S. Pat. Nos. 5,268,486; 5,650,334. Techniques for linking a preferred Cy5 fluorescent label to antibodies are described in a technical bulletin identified as Cat. No. A25000, published by Biological Detection Systems, Inc., Pittsburgh, Pa.

In Step (e), the probe is washed 1-5 times, preferably 1-3 times in a wash vessel containing a wash solution. The wash solution typically contains a buffer and a surfactant such as Tween 20.

In step (f), the probe stays in the wash vessel or is moved to a measurement vessel and the fluorescent signal of the bound immunocomplex is detected by the fluorescent detection system as described above, where the light source and the detector are mounted at the same side (the proximal side) of the sensing surface of the probe. The measurement vessel can be a separate well or can be the same pre-read vessel.

Alternatively, the methods of the present invention can be detected by other suitable fluorescent detection systems.

The analyte concentration in the sample is determined by measuring the fluorescent signal of the immunocomplex at the probe tip, subtracting the pre-read fluorescent signal of (b), and then quantitating against a calibration curve (a standard curve).

The calibration curve is typically pre-established before assaying the samples according to the methods known to a person skilled in the art. In one embodiment, the fluorescent signals (after subtracting the pre-read signal) of the same sample remain constant at each cycle, and the calibration curves are the same for each cycle. In another embodiment, the fluorescent signals (after subtracting the pre-read signal) of the same sample increase or decrease at each cycle, and a cycle-specific calibration curve needs to be established for each cycle. In these instances with changes in fluorescent signals, samples are quantitated against a cycle-specific calibration curve, and the quantitated results are shown to be consistent in spite of the increase or decrease of the fluorescent signals at different cycles.

In step (g), the probe is regenerated by employing a denaturing condition that dissociates the immune complexes bound to the capture antibody on a solid phase, but does not denature or dissociate the capture antibody from the solid phase to a degree that affects the assay performance. In general, an acid or an acidic buffer having pH about 1 to about 4 is effective to regenerate the antibody probe of the present invention. For example, hydrochloric acid, sulfuric acid, nitric acid, acetic acid can be used to regenerate the probe. The probe is first treated with an acidic condition, and then neutralized by a neutral aqueous solution such as a buffer having pH between 6.0-8.5. In one embodiment, the low pH treated probe is conveniently neutralized in the read vessel of step (b) before pre-read. Alternatively, the low pH treated probe can be neutralized in a separate vessel having a buffer with a pH of 6.0-8.5. The regeneration procedures can be one single acidic treatment, followed by neutralization. For example, a single pH 1-3, or pH 1.5-2.5 (e.g., pH 2) exposure ranging from 10 seconds to 2 minutes is effective. The regeneration procedures can also be a "pulse" regeneration step, where the probe is exposed to 2-5 cycles (e.g. 3 cycles) of a short pH treatment (e.g., 10-20 seconds), followed by neutralization at pH 6.5-8.0 (e.g., 10-20 seconds).

After regeneration of the probe, steps of (b)-(g) are repeated with a different sample in a subsequent cycle, for 1-10, 1-20, 1-25, 3-20, 5-20, 5-25, or 5-30 times, with the same probe and the same reagents.

In one embodiment, the reaction is accelerated by agitating or mixing the solution in the vessel. For example, a flow such as a lateral flow or an orbital flow of the solution across the probe tip can be induced in one or more reaction vessels, including sample vessel, reagent vessel, wash vessels, and regeneration vessel, to accelerates the binding reactions, dissociation. For example, the reaction vessels can be mounted on an orbital shaker and the orbital shaker is rotated at a speed at least 50 rpm, preferably at least 200 rpm or at least 500 rpm, such as 50-200 or 500-1,500 rpm. Additionally, the probe tip can be moved up and down and perpendicular to the plane of the orbital flow, at a speed of 0.01 to 10 mm/second, in order to induce additional mixing of the solution above and below the probe tip.

### Detecting An Analyte Having A Wide Concentration Range In A Regeneration Protocol

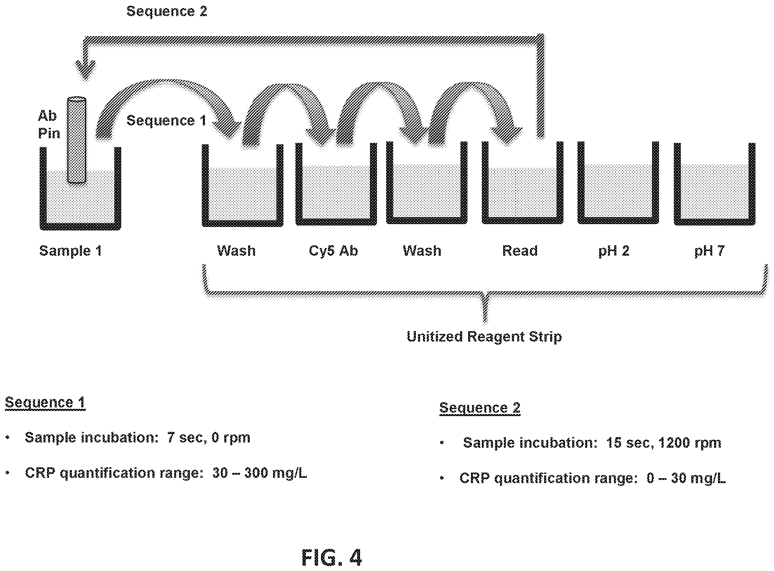

In one embodiment, the present recycle method as described above is modified to add a second sequence of binding events for quantitating an analyte that has a wide range concentration in a single assay without having to dilute the sample and repeating the assay. In this embodiment, each cycle of the immunoassay has two sequences of events each including sample binding to probe, binding reactions, and detection. In general, the assay conditions of the first sequence are optimized for samples at the high concentration end of the relevant clinical range, and the assay conditions of the second sequence are optimized for low concentration end of the relevant clinical range. After the first sequence of binding and detecting, the probe is re-dipped into the same sample vessel to bind additional analyte in the sample vessel to the probe in a more favorable binding condition (e.g., longer reaction time and/or agitation) than the binding condition in the first cycle (see FIG. 4). The analyte concentration is detected in both cycles, and the combined results provide the ability of quantitating an analyte that has a wide range concentration in a single assay without having to dilute the sample and re-do the assay.

The combined recycling and wide-range protocol comprises the steps of: (i) obtaining a probe having a first antibody immobilized on the tip of the probe, wherein the diameter of the tip surface is ≤ 5 mm; (ii) dipping the probe in a pre-read vessel comprising an aqueous solution to pre-read the fluorescent signal of the probe tip, (iii) dipping the probe tip into a first sample vessel containing a first sample solution having an analyte (for example, for 10 seconds to 2 minutes and flowing the sample solution in the sample vessel at 0-500 rpm) to bind the analyte to the first antibody on the probe tip; (iv) dipping the probe tip into a reagent vessel containing a reagent solution comprising a second antibody conjugated with fluorescent labels, to form an immunocomplex of the analyte, the first antibody, and the second antibody, wherein the first antibody and the second antibody are antibodies against the analyte; (v) dipping the probe tip into a washing vessel containing a wash solution to wash the probe tip; (vi) measuring a first fluorescent signal of the first immunocomplex formed on the probe tip; (vii) dipping the probe tip into the same sample vessel for a time period longer than that in step (iii) (for example, 1-30 minutes), and flowing the sample solution in the first sample vessel (at 0-1200 rpm, preferably 200-1200 rpm or 200-1000 rpm), to bind additional analyte in the first sample to the first antibody on the probe tip; (viii) repeating step (iv) with a longer incubation time and repeating step (v); (ix) measuring a second fluorescent signal of the second immunocomplex formed on the probe tip; and (x) determining the analyte concentration in the first sample by first subtracting the pre-read fluorescent signal of (b) from the first and second fluorescent signals, and then quantitating the analyte concentration against a high-end calibration curve or a low-end calibration curve; (xi) dipping the probe tip in an acidic solution having pH about 1.0-4.0 to elute the immunocomplex from the probe tip, and (xii) repeating steps (ii)-(xi) with a second liquid sample in a second sample vessel, whereby the analyte in multiple liquid samples is detected. The method uses the same probe, the same reagent solution, and the same washing solution in all cycles of reaction.

In the above method, steps (iii)-(vi) are the first sequence of binding events for binding an analyte having a high concentration. Steps (vii) and (viii) are the second sequence of binding events for binding an analyte having a high concentration. After the two sequences of events and measurements, the probe tip is then regenerated by steps (xi) and (ii), and then steps (iii)-(x) are repeated for the next cycle for quantitate a next sample. Unless otherwise specified, the reagents and wash solutions and procedures are the same or similar to those described in the recycling/regeneration protocol above.

### Unitized Immunoassay Strips

The present invention is further directed to a cartridge (a strip) for an immunoassay test. This unitized cartridge can be used for 2-20, or 3-20 cycles to measure 2-20, or 3-20 different samples. The cartridge comprises (a) a probe well comprising a probe and a cap, the cap being in a closed position to enclose the probe in the probe well, wherein the probe has a bottom tip coated with a first antibody; (b) a sample well to receive a sample; (c) a reagent well; (d) one or more wash wells each containing a wash solution; (e) a low pH well to provide pH of 1-4, (f) a neutralization well to provide a buffer having pH 6.0-8.5; and (g) a measurement well (a read well) having a light transmissive bottom, the measurement well containing an aqueous solution; wherein the openings of the sample well, reagent well, measurement well and wash wells are sealed. In one embodiment, the neutralization well and a measurement well (a read well) are the same well. In another embodiment, the neutralization well and a measurement well are two separate wells.

The cartridge is similar to that described in U.S. Patent No. 8,753,574; except that the cartridge of this invention contains additional low pH well and neutralization well.

A sample well is a well that receives a sample containing an analyte. A sample well can be a blank well, or it can contain detergents, blocking agents and various additives for the immunoassay, either in a dry format or in a wet (liquid) format.

A reagent well contains reagents such as a fluorescent labelled antibody that reacts with the analyte to form an immunocomplex and generate a signal for detection. The reagents can be in a wet format or in a dry format. The wet format contains a reagent in an assay buffer. The wet format is typically in a small liquid volume (< 10 µL, e.g., 5 µL). An assay buffer typically includes a buffer (e.g., phosphate, tris), a carrier protein (e.g., bovine serum albumin, porcine serum albumin, and human serum albumin, 0.1-50 mg/mL), a salt (e.g., saline), and a detergent (e.g., Tween, Triton). An example of an assay buffer is phosphate buffered saline, pH 7.4, 5 mg/ml bovine serum albumin, 0.05% Tween 20. The assay buffer optionally contains a blocking agent in an amount of 1-500 µg/mL. The final formulation will vary depending on the requirements of each analyte assay. The dry format is the dry form of the reagent in an assay buffer. The dry format includes lyophilization cake, powder, tablet or other formats typical in diagnostic kits. The dry format is to be reconstituted to a wet format by a reconstitution buffer or a wash buffer.

The cartridge comprises one or more washing wells each containing an aqueous solution. The wash wells contain a wash buffer to wash the probe after binding steps in the sample well and reagent well. One to four wash wells (e.g., 1, 2, 3, or 4 wells) are dedicated for washing after each binding step. Wash buffers contain detergents. Any detergent typically used in immunoassays (e.g., Tween, Triton) can be used in this invention.

The cartridge comprises a measurement well having an optically clear bottom that enables the detection of the labeled-immunocomplex bound to the bottom tip of the probe. The measurement is through the bottom of the well.

In one embodiment, the cartridge further comprises one or more reconstitution wells that contain reconstitution buffer to be dispensed into the sample well and reagent well to reconstitute the dry forms in the sample well and reagent well. The reconstitution buffer can be simply a buffer such as phosphate-buffer saline. The reconstitution buffer can additionally include other additives (carrier protein, blockers, detergents, etc.) contained in the assay buffer.

The openings of the reagent well and wash well(s) are sealed with a foil or a film. The seal is penetrable. The wells may be opened by piercing the seal by a manual or automated device. In one embodiment, when the cap of the probe is in a closed position, the cap is folded over the probe well to enclose the probe in the probe well, but the cap does not cover the sample well, the wash wells or the measurement well.

### Probe Comprising an Immobilized Antibody

The present invention provides a probe comprising an antibody immobilized on the tip of the probe, wherein the antibody is labelled with biotin and is indirectly immobilized on the probe tip by streptavidin coated on the probe tip; wherein the diameter of the tip surface is ≤ 5 mm, and the antibody does not substantially denature or dissociate from the probe after an acidic treatment; i.e., no more than 15%, preferably no more than 10% or 5% of the antibody is denatured or dissociated from the probe after 1-20 cycles of the acid treatment. The acid treatment is typically performed by dipping the probe in a low pH buffer (pH 1-4, or 1-3, or 1.5-2.5) for 10 seconds to 2 minutes.

The probe comprising a captured antibody as described above is useful for an immunoassay because the probe survives an acid regeneration and yields consistent dose response curves for at least 20 regeneration cycles. This probe needs only a single calibration curve for all 20 cycles. On the contrary, a probe comprising a capture antibody whose binding activity changes after acid regeneration will require a cycle specific calibration curve for every cycle, which adds to the cost and time for an assay, and it is more work in order to maintain a consistent performance throughout the cycles.

The inventor has discovered that mouse monoclonal anti-human C-reactive protein C30 antibody (an IgG1 isotype) from HyTest (Turku, Finland), when labeled with biotin and immobilized onto the probe tip through streptavidin coated on the probe tip, and used in the present method, the fluorescent signal at the completion of each cycle of reaction after subtracting the pre-read signal, remains constant for 20 cycles of reaction using the same probe and the same reagents.

The invention is illustrated further by the following examples that are not to be construed as limiting the invention in scope to the specific procedures described in them.

### EXAMPLES

### Example 1. Preparing Antibody-Coated Probe

Quartz probes, 1 mm diameter and 2 cm in length, were coated with aminopropylsilane using a chemical vapor deposition process (Yield Engineering Systems, 1224P) following manufacturer's protocol. The probe tip was then immersed in a solution of streptavidin (Sigma-Aldrich), 10 ug/ml in phosphate buffered saline pH 7.4 (PBS). After allowing the protein to adsorb to the probe for 5 minutes, the probe tip was washed in PBS. The probe tip was then immersed in a solution containing a biotin labeled antibody at 10 µg/ml in PBS. After 10 minutes the probe tip was washed in PBS. The antibodies were biotinylated by a standard method. The biotinylated antibodies were designated as "capture antibody".

### Example 2. Preparing Cy5 Labeled Antibody

Antibody at 3.2 mg/ml in 1 ml 0.1 M sodium carbonate pH 9.5 was mixed with 10.6 µl Cy5-NHS (GE Healthcare) at 10 mg/ml DMF and allowed to reacted for ½ hour at 30C. The mixture was then purified on a PD 10 column (GE Healthcare). The Cy5 labeled antibodies were designated as "signal antibody".

### Example 3. C-reactive Protein Immunoassay Protocol

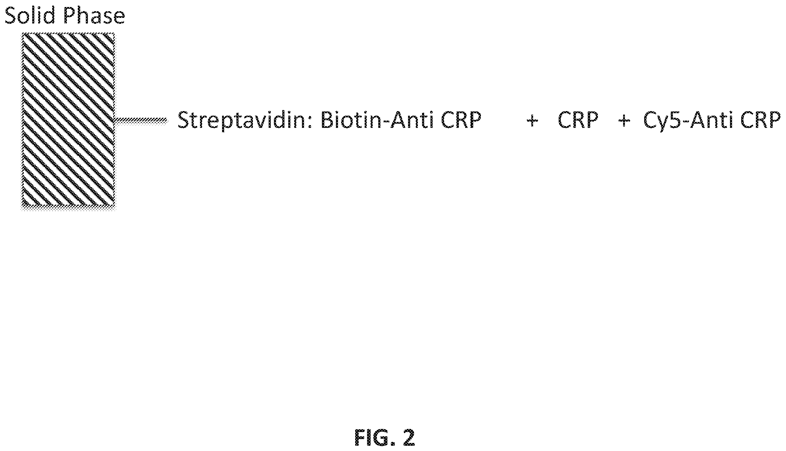

FIG. 2 shows the basic assay format consisting of a biotinylated anti C-reactive protein (CRP) antibody bound to streptavidin immobilized to the probe tip. The streptavidin in this case serves a spacer preventing the antibody from interacting directly with the probe surfaces, potentially interfering with its binding activity.

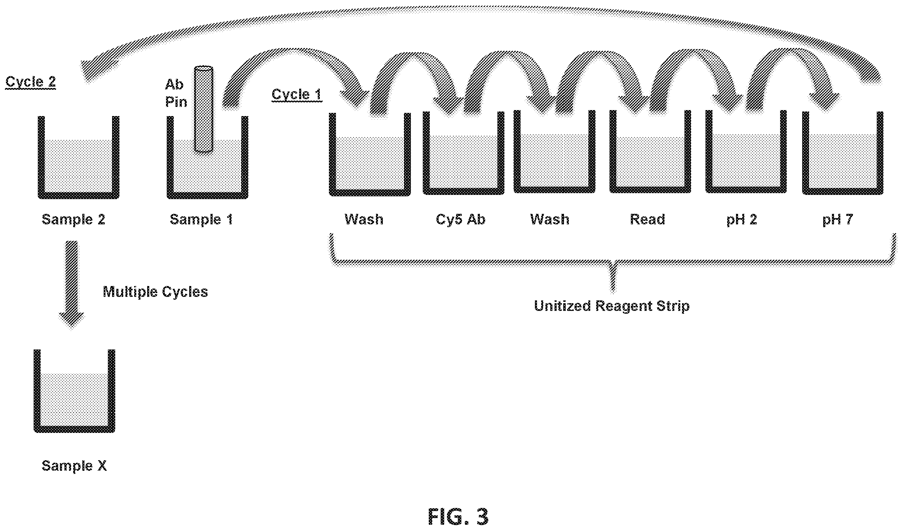

Figure 3 is a schematic of an assay protocol. After incubation in sample, the antibody (Ab)-coated probe is transferred to a wash well and followed by incubation with the Cy5 labeled second antibody. After the incubation with the second Ab, a wash cycle is carried out, then the fluorescence is measured at the probe tip to complete the assay. Immersion of the probe in a low pH buffer, pH 2, dissociates the CRP immune complex and immersion in a pH 7 buffer conditions the probe for a subsequent sample analysis. The streptavidin:biotin-Ab complex remains intact on the probe tip during the low pH exposure. Typically, much harsher denaturation conditions, such as 8M urea or 6M guanidine, are required to disassociate biotin from streptavidin.

In a subsequent sample analysis, the probe and all the reagents to perform the assay are re-used.

### Example 4. C-reactive Protein Immunoassay (Wide Concentration Range)

Figure 4 illustrates the probe transfer sequence in a "wide concentration range" protocol, which can quantitate a wide range of analyte concentration (e.g., 30-300mg/L CRP) in a single assay. Sequence 1 quantitates analyte having high concentration (30-300 mg/L) and sequence 2 quantitates samples with low concentration (0-30 mg/L). The combined quantitation provides the quantitation over a wide range of over 100-fold concentration.

Three sets of samples were measured for fluorescent signals by the following protocol. Set 1 has 20 different samples each having a CRP concentration of 30 mg/L in assay buffer (0.5mg/mL bovine serum albumin (BSA), phosphate-buffered saline, 0.05% Tween 20, pH 7.4). Set 2 has 20 different samples each having a CRP concentration of 100 mg/L in assay buffer. Set 3 has 20 different samples each having a CRP concentration of 300 mg/L in assay buffer. The same reagents were used for 20 cycles of measurements of each set of 20 different samples.

### Sequence 1 (high concentration detection)

1. Pre-read
2. First sample (CRP) incubation: 7 seconds 0 RPM
3. Three wash: 7 seconds 1200 RPM
4. Cy5_C5 incubation: 7 seconds 1200 RPM
5. Three wash: 7 seconds 1200RPM
6. 1^{st} read

### Sequence 2 (low concentration detection)

7. Same first sample (CRP) incubation : 15 seconds 1200 RPM
8. Three wash: 7 seconds 1200 RPM
9. Cy5_C5 incubation: 15 seconds 1200 RPM
10. Three wash: 15 seconds 1200 RPM
11. 2^{nd} read

### Pulse Regeneration

12. Regeneration buffer (pH 2.0): 10 sec 500 RPM
13. PBS (pH 7.4): 10 sec 500 RPM
14. Repeat 12
15. Repeat 13
16. Repeat 12
17. Repeat 13
18. Return to 1, for subsequent cycles for different samples

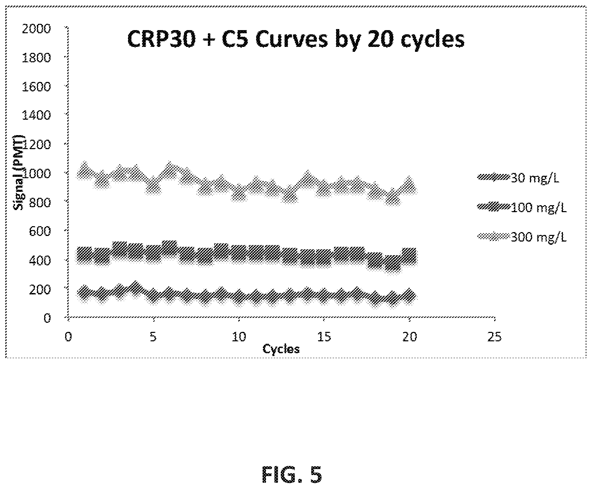

Hytest mouse anti-human CRP monoclonal antibody C30 was used as capture antibody and Hytest mouse anti-human CRP monoclonal antibody C5 was used for signal antibody. Figure 5 shows the results of CRP assays of the three sets of samples (Sequence 1, n=20 in each set); the results show that the fluorescent signals are consistent with low coefficient of variation (CV). The fluorescent signals are summarized in Table 1.

**Table 1.**

| CRP (mg/L) | Signal Average | Signal SD | CV (%) |
|---|---|---|---|
| 30 | 155 | 16 | 10 |
| 100 | 424 | 22 | 5 |
| 300 | 937 | 54 | 6 |

### Example 5. C-reactive Protein Immunoassay (Wide Concentration Range Protocol)

Six sets of samples were measured for fluorescent signals by the same protocol in Example 4. Each set have PBS samples with CRP concentration of 0, 3, 10, 30, 100, or 300 mg/L. Each sample was measured 9 times in 9 cycles. The same reagents were used for 9 cycles of measurements of each set of samples.

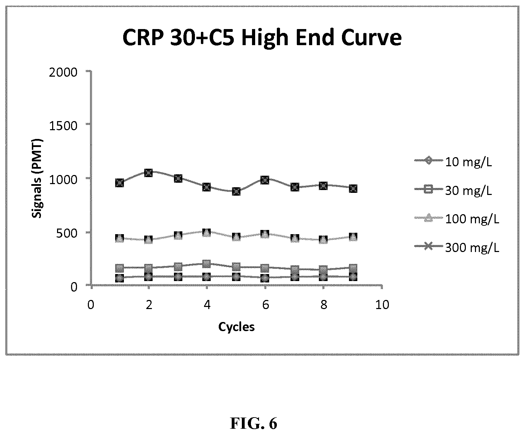

The first step in this protocol is a "pre-read" to measure the background fluorescence associated with the probe. Table 2 shows the "pre-read: signals taken before each cycle from the CRP samples. Pre-read data indicate that the acid elution is not complete with residual fluorescence on the probe after each cycle and before next cycle. The results show that the fluorescent signal in the "pre-reads" increases with each cycle, however, subtraction of the "pre-reads" yield the consistent results at each cycle as shown in FIGs. 5 and 6.

**Table 2 (Pre-Read Signals)**

| CRP(mg/L) | Cycles | | | | | | | | | |
|---|---|---|---|---|---|---|---|---|---|---|
| | 1 | 2 | 3 | 4 | 5 | 6 | 7 | 8 | 9 | 10 |
| 0 | 97 | 121 | 156 | 175 | 195 | 212 | 229 | 244 | 259 | 282 |
| 1 | 153 | 196 | 237 | 265 | 288 | 310 | 333 | 351 | 369 | 388 |
| 3 | 95 | 115 | 150 | 173 | 195 | 215 | 234 | 246 | 259 | 277 |
| 10 | 95 | 122 | 160 | 190 | 217 | 240 | 261 | 281 | 300 | 321 |
| 30 | 91 | 138 | 195 | 238 | 238 | 277 | 310 | 339 | 366 | 417 |
| 100 | 84 | 147 | 210 | 257 | 306 | 351 | 393 | 430 | 470 | 508 |
| 300 | 93 | 147 | 209 | 262 | 308 | 352 | 393 | 434 | 474 | 513 |

The fluorescent signals of high-end curve (Sequence 1, n=9) are shown in Figure 6 and Table 3.

**Table 3.**

| CRP (mg/L) | Signal Average | Signal SD | CV (%) |
|---|---|---|---|
| 10 | 80 | 4 | 5 |
| 30 | 168 | 15 | 9 |
| 100 | 453 | 25 | 5 |
| 300 | 954 | 55 | 6 |

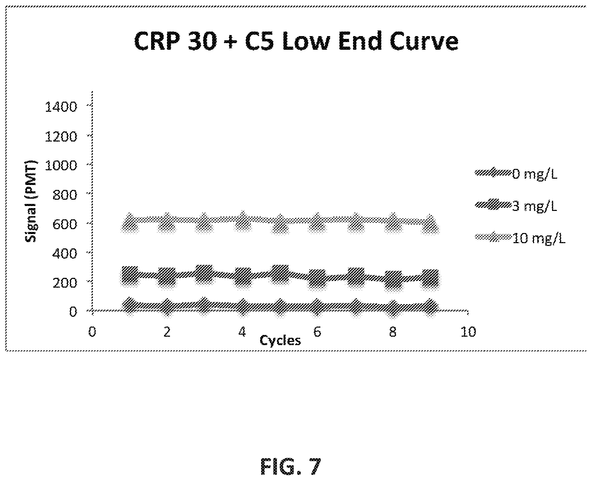

The fluorescent signals of low-end curve (Sequence 2, n=20) are shown in Figure 7 and Table 4.

**Table 4.**

| CRP (mg/L) | Signal Average | Signal SD | CV (%) |
|---|---|---|---|
| 0 | 30 | 5 | |
| 3 | 231 | 14 | 6 |
| 10 | 617 | 8 | 1 |

Figure 6 depicts the assay signals of high CRP samples from 10 to 300 mg/L and Figure 7 shows second measurements of assay signals of low CRP samples from 0 to 10mg/L. Assay signals were consistent out for 9 cycles for all CRP levels measured.

### Example 6. Measuring CRP in High Concentration to Low Concentration Sample

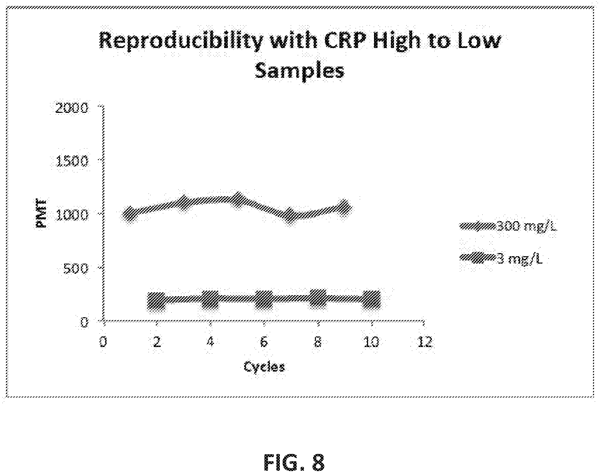

In clinical practice, CRP samples are assayed in a random sequence. To evaluate whether the assay of a very high sample could bias results of a subsequent low sample, we assayed 10 cycles of a 300 mg/L sample followed by a 3 mg/L sample. The 300 mg/L is at the top of the quantification range representing a CRP level associated with extreme inflammation, while 3 mg/L is within the normal range. Figure 8 shows the results of CRP assays of the two sets of samples; the results show that the fluorescent signals (after subtracting pre-read signal) are consistent for both 300 mg/L and 3 mg/L, with negligible bias alternating between the high and low samples. The fluorescent signals are summarized in Table 5.

**Table 5.**

| CRP (mg/L) | Average (n=5) | SD | CV (%) |
|---|---|---|---|
| 3 | 202 | 8 | 4 |
| 300 | 1051 | 69 | 7 |

### Example 7. Comparison Study

This experiment compares the CRP results of 100 clinical samples measured by the present protocol as shown in Example 4, and by an established clinical instrument, the Siemens BN II.

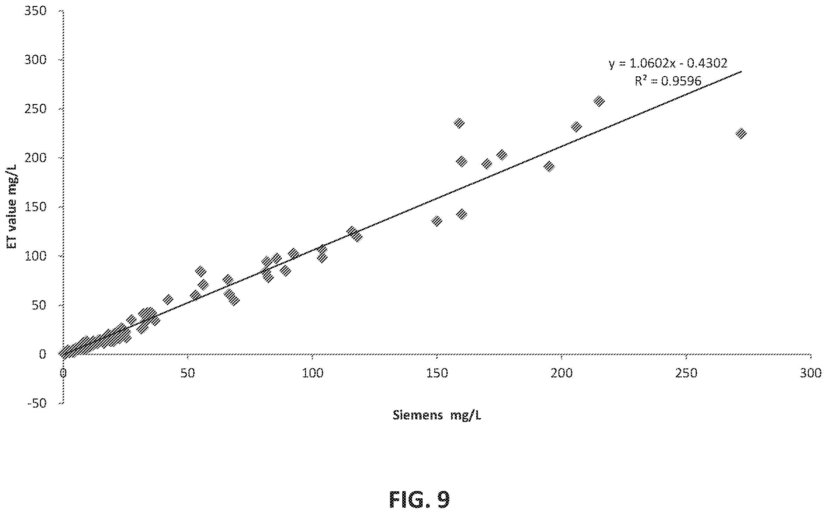

100 samples were quantified by the present method using 10 test strips. Each strip assayed 10 randomly selected samples in 10 cycles of re-using the same test probe. The Siemens results were obtained following the standard protocol from the manufacture. The result comparison of the present method vs. Siemens method is shown in FIG. 9, which shows that the results generated by the present method are highly correlated to those generated by the Siemens method with R² being 0.9596 (R= correlation coefficient).

### Example 8. CRP Assay with a Different Capture Antibody

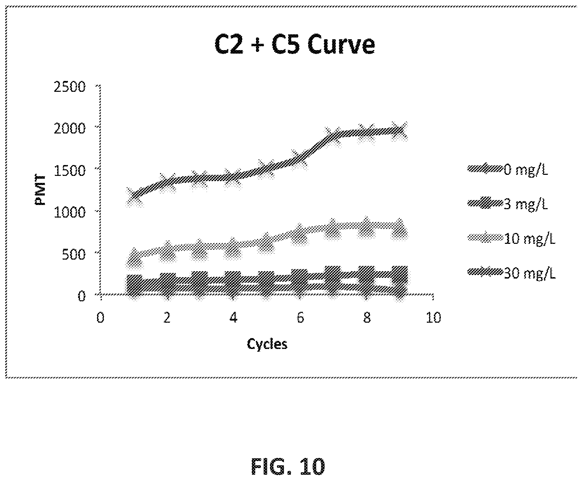

A second capture antibody (Hytest mouse monoclonal anti-human CRP C2) was evaluated in the same protocol as described in Example 4. Figure 10 shows the unexpected result in the CRP signals increase with each cycle, even after subtraction of pre-reads. The acid treatment could alter the protein on the surface of the probe so that either the antibody binding capacity increases or the fluorescence signal is altered. Fluorescence is known to be very sensitive to environmental effects.

In order to address the increase in fluorescence signal at each cycle, a standard curve is established in each cycle, and the quantitation of analyte in each cycle is calculated against the cycle-specific standard curve. A CRP sample of 6.0 mg/mL in assay buffer was tested over 9 cycles and quantitated against cycle-specific standard curve to determine reproducibility. The results show an average of 6.1 mg/mL, with standard deviation of 0.5 mg/mL, and CV of 9%.

In spite of the increase in fluorescence signal at each cycle, consistent quantification was achieved by running a calibration curve for each cycle.

### Example 9. Procalcitonin Assay

The following protocol lists the steps for the assays of procalcitonin (PCT).

### Sequence 1 (high concentration detection)

1. Pre-read
2. First sample (PCT) incubation: 15 seconds 0 RPM
3. Three wash: 7 seconds 1200 RPM
4. Cy5_16B5 incubation: 15 seconds 1200 RPM
5. Three wash: 7 seconds 1200RPM
6. 1^{st} read

### Sequence 2 (low concentration detection)

7. First sample (PCT) incubation : 360 seconds 1200 RPM
8. Three wash: 7 seconds 1200 RPM
9. Cy5_16B5 incubation: 60 seconds 1200 RPM
10. Three wash : 15 seconds 1200 RPM
11. 2^{nd} read

### Regeneration

12. Regeneration buffer (pH 2.0): 10 sec 500 RPM
13. PBS (pH 7.4): 10 sec 500 RPM
14. Return to 1 , for a subsequent cycle on a different sample

The PCT protocol is similar to that of CRP (see Example 4), except that steps 2 and 7 have longer incubations to account for the much lower concentrations of PCT. Also, the PCT protocol only uses a single 10 second, pH 2.0 regeneration. The capture antibody is a goat polyclonal anti-PCT (Hytest, PPC3) and the signal antibody is a monoclonal anti-PCT (Hytest, 16B5).

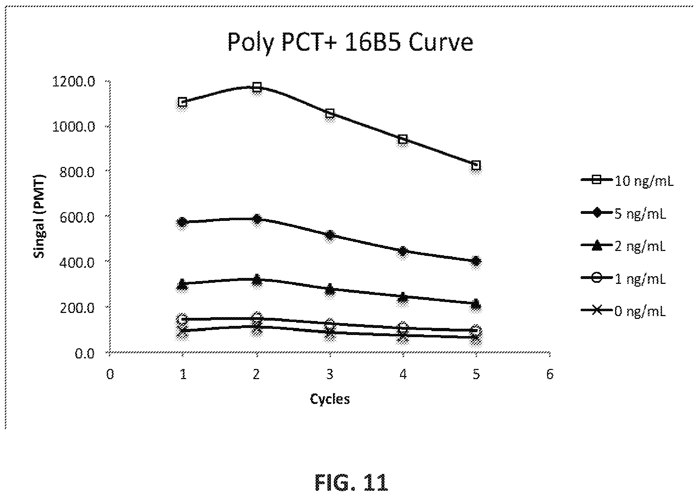

The fluorescent signals of 5 cycles are summarized in Figure 11, which show a different effect to the recycle protocol compared to the two CRP capture antibodies. By 5 cycles, the fluorescent signals show a slight decline.

In order to address the decrease in fluorescence signal at each cycle, a standard curve is established for each cycle, and the quantitation of analyte in each cycle is calculated against the cycle-specific standard curve. A PCT sample of 5.0 ng/mL in assay buffer was tested over 5 cycles and quantitated against cycle-specific standard curve to determine reproducibility. The results show an average of 4.8 ng/mL, with standard deviation of 0.4 ng/mL, and CV of 9%.

In spite of the decrease in fluorescence signal at each cycle, consistent quantification was achieved by running a calibration curve for each cycle.

The invention, and the manner and process of making and using it, are now described in such full, clear, concise and exact terms as to enable any person skilled in the art to which it pertains, to make and use the same. It is to be understood that the foregoing describes preferred embodiments of the present invention and that modifications may be made therein without departing from the scope of the present invention as set forth in the claims. To particularly point out and distinctly claim the subject matter regarded as invention, the following claims conclude this specification

## Claims

1. A method of detecting c-reactive protein in multiple liquid samples, comprising the steps of:
(a) obtaining a probe having a first antibody immobilized on the tip of the probe, wherein the diameter of the tip surface is ≤ 5 mm; wherein the first antibody is a mouse monoclonal anti-human C-reactive protein antibody CRP30.
(b) dipping the probe in a pre-read vessel comprising an aqueous solution having pH of 6.0-8.5 to pre-read the fluorescent signal of the probe tip;
(c) dipping the probe tip into a sample vessel containing a liquid sample having an analyte of c-reactive protein;
(d) dipping the probe tip into a reagent vessel containing a reagent solution comprising a second antibody conjugated with one or more fluorescent labels to form an immunocomplex among the analyte, the first antibody, and the second antibody on the probe tip, wherein the first antibody and the second antibody are antibodies against the analyte;
(e) dipping the probe tip into a washing vessel containing a wash solution;
(f) determining the analyte concentration in the first sample by measuring the fluorescent signal of the immunocomplex at the probe tip, subtracting the pre-read fluorescent signal of (b), and quantitating against a calibration curve;
(g) dipping the probe tip in an acidic solution having pH about 1.0-4.0 to elute the immunocomplex from the probe tip; and
(h) repeating steps (b)-(g) with a second liquid sample in a second sample vessel in a second cycle, whereby the analyte in multiple liquid samples is detected.

2. The method of Claim 1, wherein the calibration curves in step (f) are the same for all cycles of quantitation.

3. The method of Claim 1, wherein the acidic solution in step (g) has a pH of 1.5-2.5.

4. The method of Claim 1, where in step (g), the probe tip is exposed to the acidic solution one time for 10 second to 2 minutes.

5. The method of Claim 1, where in step (g), the probe tip is exposed to a pulse treatment of 2-5 cycles of the acidic solution treatment followed by neutralization in the read vessel for 10-20 seconds.

6. A method of detecting c-reactive protein having a wide concentration range in multiple liquid samples, comprising the steps of:
(i) obtaining a probe having a first antibody immobilized on the tip of the probe, wherein the diameter of the tip surface is ≤ 5 mm; wherein the first antibody is a mouse monoclonal anti-human C-reactive protein antibody CRP30.
(ii) dipping the probe in a pre-read vessel comprising an aqueous solution to pre-read the fluorescent signal of the probe tip,
(iii) dipping the probe tip into a first sample vessel containing a first sample solution having an analyte of c-reactive protein to bind the analyte to the first antibody on the probe tip;
(iv) dipping the probe tip into a reagent vessel containing a reagent solution comprising a second antibody conjugated with fluorescent labels, to form an immunocomplex of the analyte, the first antibody, and the second antibody, wherein the first antibody and the second antibody are antibodies against the analyte;
(v) dipping the probe tip into a washing vessel containing a wash solution to wash the probe tip;
(vi) measuring a first fluorescent signal of the first immunocomplex formed on the probe tip;
(vii) dipping the probe tip into the same sample vessel for a time period longer than that in step (iii), and flowing the sample solution in the first sample vessel, to bind additional analyte in the first sample to the first antibody on the probe tip;
(viii) repeating step (iv) with a longer incubation time and repeating step (v);
(ix) measuring a second fluorescent signal of the second immunocomplex formed on the probe tip;
(x) determining the analyte concentration in the first sample by first subtracting the pre-read fluorescent signal of (b) from the first and second fluorescent signals, and then quantitating the analyte concentration against a high-end calibration curve or a low-end calibration curve;
(xi) dipping the probe tip in an acidic solution having pH about 1.0-4.0 to elute the immunocomplex from the probe tip, and
(xii) repeating steps (ii)-(xi) with a second liquid sample in a second sample vessel in a second cycle, whereby the analyte in multiple liquid samples is detected.

7. The method of Claim 6, wherein the high end and the low end calibration curves in step (x) are the same for all cycles of quantitation.

8. The method of Claim 6, wherein the high end and the low end calibration curves in step (x) are cycle-specific calibration curves.

9. The method of Claim 6, wherein the acidic solution in step (xi) has a pH of 1.5-2.5.

10. The method of Claim 6, where in step (xi), the probe tip is exposed to the acidic solution one time for 10 second to 2 minutes.

11. The method of Claim 1 or 6, wherein the first antibody is labeled with biotin and is indirectly immobilized on the sensing surface coated with streptavidin.

12. A cartridge for an immunoassay of c-reactive protein, comprising (a) a probe well comprising a probe and a cap, the cap being in a closed position to enclose the probe in the probe well, wherein the probe has a bottom tip coated with a first antibody; wherein the first antibody is a mouse monoclonal anti-human C-reactive protein antibody CRP30; (b) a sample well to receive a sample; (c) a reagent well containing reagents; (d) one or more wash wells each containing a wash solution; (e) a low pH well comprising an acidic reagent to provide pH of 1-4, (f) a neutralization well containing a buffer reagent having pH 6.0-8.5; and (g) a measurement well having a light transmissive bottom, the measurement well containing an aqueous solution; wherein the openings of the sample well, reagent well, measurement well and wash wells are sealed.

13. A cartridge for an immunoassay of c-reactive protein, comprising (a) a probe well comprising a probe and a cap, the cap being in a closed position to enclose the probe in the probe well, wherein the probe has a bottom tip coated with a first antibody, wherein the first antibody is a mouse monoclonal anti-human C-reactive protein antibody CRP30; (b) a sample well to receive a sample; (c) a reagent well containing reagents; (d) one or more wash wells each containing a wash solution; (e) a low pH well containing an acidic pH reagent to provide pH of 1-4, and (f) a neutralization and measurement well having a light transmissive bottom and containing an aqueous buffer solution having pH 6.0-8.5; wherein the openings of the sample well, reagent well, measurement well and wash wells are sealed.

14. A probe comprising a monolithic substrate coated with antibody at the probe tip, wherein the probe has an aspect ratio of length to width of at least 5 to 1, the diameter of the tip surface is ≤ 5 mm, and the antibody is mouse monoclonal anti-human C-reactive protein antibody CRP30, optionally wherein the antibody is labeled with biotin and immobilized onto the probe tip by streptavidin coated on the probe tip.

## Patentansprüche

1. Verfahren zum Nachweisen von C-reaktivem Protein in mehreren flüssigen Proben, umfassend die folgende Schritte:
(a) Erhalten einer Sonde mit einem ersten Antikörper, der an der Spitze der Sonde immobilisiert ist, wobei der Durchmesser der Spitzenoberfläche ≤ 5 mm ist; wobei der erste Antikörper ein monoklonaler, auf C-reaktives Protein gerichteter Maus-Anti-Human-Antikörper CRP30 ist.
(b) Eintauchen der Sonde in ein Pre-Read-Gefäß, das eine wässrige Lösung mit einem pH-Wert von 6,0-8,5 umfasst, um das Fluoreszenzsignal der Sondenspitze vorab zu lesen;
(c) Eintauchen der Sondenspitze in ein Probengefäß, das eine flüssige Probe mit einem Analyten für C-reaktives Protein enthält;
(d) Eintauchen der Sondenspitze in ein Reagenzgefäß, das eine Reagenzlösung enthält, die einen zweiten Antikörper umfasst, der mit einer oder mehreren fluoreszierenden Markierungen konjugiert ist, um einen Immunkomplex zwischen dem Analyten, dem ersten Antikörper und dem zweiten Antikörper auf der Sondenspitze auszubilden, wobei der erste Antikörper und der zweiter Antikörper Antikörper gegen den Analyten sind;
(e) Eintauchen der Sondenspitze in ein Waschgefäß, das eine Waschlösung enthält;
(f) Bestimmen der Analytkonzentration in der ersten Probe durch Messen des Fluoreszenzsignals des Immunkomplexes an der Sondenspitze, Subtrahieren des vorab ausgelesenen Fluoreszenzsignals von (b) und Quantifizieren gegen eine Kalibrierungskurve;
(g) Eintauchen der Sondenspitze in eine saure Lösung mit einem pH-Wert von etwa 1,0-4,0, um den Immunkomplex von der Sondenspitze zu eluieren; und
(h) Wiederholen der Schritte (b)-(g) mit einer zweiten flüssigen Probe in einem zweiten Probengefäß in einem zweiten Zyklus, wobei der Analyt in mehreren flüssigen Proben nachgewiesen wird.

2. Verfahren nach Anspruch 1, wobei die Kalibrierungskurven in Schritt (f) für alle Quantifizierungszyklen gleich sind.

3. Verfahren nach Anspruch 1, wobei die saure Lösung in Schritt (g) einen pH-Wert von 1,5-2,5 aufweist.

4. Verfahren nach Anspruch 1, wobei in Schritt (g) die Sondenspitze einmalig 10 Sekunden bis 2 Minuten lang der sauren Lösung ausgesetzt wird.

5. Verfahren nach Anspruch 1, wobei in Schritt (g) die Sondenspitze einer Impulsbehandlung von 2-5 Zyklen der Behandlung mit saurer Lösung ausgesetzt wird, gefolgt von einer Neutralisation in dem Read-Gefäß für 10-20 Sekunden.

6. Verfahren zum Nachweisen von C-reaktivem Protein mit einem breiten Konzentrationsbereich in mehreren flüssigen Proben, umfassend die folgenden Schritte:
(i) Erhalten einer Sonde mit einem ersten Antikörper, der an der Spitze der Sonde immobilisiert ist, wobei der Durchmesser der Spitzenoberfläche ≤ 5 mm ist; wobei der erste Antikörper ein monoklonaler, auf C-reaktives Protein gerichteter Maus-Anti-Human-Antikörper CRP30 ist.
(ii) Eintauchen der Sonde in ein Pre-Read-Gefäß, das eine wässrige Lösung umfasst, um das Fluoreszenzsignal der Sondenspitze vorab zu lesen,
(iii) Eintauchen der Sondenspitze in ein erstes Probengefäß, das eine erste Probenlösung mit einem Analyten des C-reaktiven Proteins enthält, um den Analyten an den ersten Antikörper auf der Probenspitze zu binden;
(iv) Eintauchen der Sondenspitze in ein Reagenzgefäß, das eine Reagenzlösung enthält, die einen zweiten Antikörper umfasst, der mit fluoreszierenden Markierungen konjugiert ist, um einen Immunkomplex des Analyten, des ersten Antikörpers und des zweiten Antikörpers auszubilden, wobei der erste Antikörper und der zweiter Antikörper Antikörper gegen den Analyten sind;
(v) Eintauchen der Sondenspitze in ein Waschgefäß, das eine Waschlösung enthält, um die Probenspitze zu waschen;
(vi) Messen eines ersten Fluoreszenzsignals des ersten an der Sondenspitze ausgebildeten Immunkomplexes;
(vii) Eintauchen der Sondenspitze in dasselbe Probengefäß für einen längeren Zeitraum als in Schritt (iii) und Fließenlassen der Probenlösung in das erste Probengefäß, um noch mehr Analyt in der ersten Probe an den ersten Antikörper an der Sondenspitze zu binden;
(viii) Wiederholen von Schritt (iv) mit einer längeren Inkubationszeit und Wiederholen von Schritt (v);
(ix) Messen eines zweiten Fluoreszenzsignals des zweiten an der Sondenspitze ausgebildeten Immunkomplexes;
(x) Bestimmen der Analytkonzentration in der ersten Probe durch zuerst Subtrahieren des Pre-Read-Fluoreszenzsignals von (b) von dem ersten und zweiten Fluoreszenzsignal und dann Quantifizieren der Analytkonzentration gegen eine High-End-Kalibrierungskurve oder eine Low-End-Kalibrierungskurve;
(xi) Eintauchen der Sondenspitze in eine saure Lösung mit einem pH-Wert von etwa 1,0-4,0, um den Immunkomplex von der Sondenspitze zu eluieren; und
(xii) Wiederholen der Schritte (ii)-(xi) mit einer zweiten flüssigen Probe in einem zweiten Probengefäß in einem zweiten Zyklus, wobei der Analyt in mehreren flüssigen Proben nachgewiesen wird.

7. Verfahren nach Anspruch 6, wobei die High-End-Kalibrierungskurve und die Low-End-Kalibrierungskurve in Schritt (x) für alle Quantifizierungszyklen gleich sind.

8. Verfahren nach Anspruch 6, wobei die High-End-Kalibrierungskurve und die Low-End-Kalibrierungskurve in Schritt (x) zyklusspezifische Kalibrierungskurven sind.

9. Verfahren nach Anspruch 6, wobei die saure Lösung in Schritt (xi) einen pH-Wert von 1,5-2,5 aufweist.

10. Verfahren nach Anspruch 6, wobei in Schritt (xi) die Sondenspitze einmalig 10 Sekunden bis 2 Minuten lang der sauren Lösung ausgesetzt wird.

11. Verfahren nach Anspruch 1 oder 6, wobei der erste Antikörper mit Biotin markiert ist und indirekt auf der mit Streptavidin beschichteten Abtastoberfläche immobilisiert wird.

12. Kartusche für einen Immunoassay auf C-reaktives Protein, umfassend (a) ein Sonden-Well, das eine Sonde und eine Kappe umfasst, wobei sich die Kappe in einer geschlossenen Position befindet, um die Sonde in dem Sonden-Well einzuschließen, wobei die Sonde eine mit einem ersten Antikörper beschichtete untere Spitze aufweist; wobei der erste Antikörper ein monoklonaler, auf C-reaktives Protein gerichteter Maus-Anti-Human-Antikörper CRP30 ist; (b) ein Proben-Well, um eine Probe aufzunehmen; (c) ein Reagenz-Well, das Reagenzien enthält; (d) ein oder mehrere Wasch-Wells, die jeweils eine Waschlösung enthalten; (e) ein Well mit niedrigem pH-Wert, umfassend ein saures Reagenz, um einen pH-Wert von 1-4 bereitzustellen, (f) ein Neutralisierungs-Well, das ein Pufferreagenz mit einem pH-Wert von 6,0-8,5 enthält; und (g) ein Mess-Well mit einem lichtdurchlässigen Boden, wobei das Mess-Well eine wässrige Lösung enthält; wobei die Öffnungen des Proben-Wells, des Reagenz-Wells, des Mess-Wells und der Wasch-Wells abgedichtet sind.

13. Kartusche für einen Immunoassay auf C-reaktives Protein, umfassend (a) ein Sonden-Well, das eine Sonde und eine Kappe umfasst, wobei sich die Kappe in einer geschlossenen Position befindet, um die Sonde in dem Sonden-Well einzuschließen, wobei die Sonde eine mit einem ersten Antikörper beschichtete untere Spitze aufweist, wobei der erste Antikörper ein monoklonaler, auf C-reaktives Protein gerichteter Maus-Anti-Human-Antikörper CRP30 ist; (b) ein Proben-Well, um eine Probe aufzunehmen; (c) ein Reagenz-Well, das Reagenzien enthält; (d) ein oder mehrere Wasch-Wells, die jeweils eine Waschlösung enthalten; (e) ein Well mit niedrigem pH-Wert, das ein Reagenz mit saurem pH-Wert enthält, um einen pH-Wert von 1-4 bereitzustellen, und (f) ein Neutralisierungs- und Mess-Well mit einem lichtdurchlässigen Boden und einer wässrigen Pufferlösung mit einem pH-Wert von 6,0-8,5; wobei die Öffnungen des Proben-Wells, des Reagenz-Wells, des Mess-Wells und der Wasch-Wells abgedichtet sind.

14. Sonde, umfassend ein monolithisches Substrat, das an der Sondenspitze mit einem Antikörper beschichtet ist, wobei die Sonde ein Seitenverhältnis von Länge zu Breite von wenigstens 5 zu 1 aufweist, der Durchmesser der Spitzenoberfläche ≤ 5 mm ist und der Antikörper ein monoklonaler, auf C-reaktives Protein gerichteter Maus-Anti-Human-Antikörper CRP30 ist, wobei der Antikörper optional mit Biotin markiert ist und durch Streptavidin, das auf die Sondenspitze aufgetragen ist, auf der Sondenspitze immobilisiert wird.

## Revendications

1. Procédé de détection d'une protéine c-réactive dans plusieurs échantillons liquides, comprenant les étapes :
(a) d'obtention d'une sonde ayant un premier anticorps immobilisé sur la pointe de la sonde, le diamètre de la surface de pointe étant ≤ 5 mm ; le premier anticorps étant un anticorps monoclonal de souris anti-protéine c-réactive humaine CRP30 ;
(b) de trempage de la sonde dans un récipient de prélecture comprenant une solution aqueuse ayant un pH de 6,0 à 8,5 pour prélire le signal fluorescent de la pointe de sonde ;
(c) de trempage de la pointe de sonde dans un récipient à échantillon contenant un échantillon liquide ayant un analyte de protéine c-réactive ;
(d) de trempage de la pointe de sonde dans un récipient de réactif contenant une solution de réactif comprenant un second anticorps conjugué avec un ou plusieurs marqueurs fluorescents pour former un complexe immunitaire entre l'analyte, le premier anticorps et le second anticorps sur la pointe de sonde, le premier anticorps et le second anticorps sont des anticorps contre l'analyte ;
(e) de trempage de la pointe de sonde dans un récipient de lavage contenant une solution de lavage ;
(f) de détermination de la concentration d'analyte dans le premier échantillon en mesurant le signal fluorescent de l'immunocomplexe au niveau de la pointe de sonde, en soustrayant le signal fluorescent prélu de (b), et en quantifiant par rapport à une courbe d'étalonnage ;
(g) de trempage de la pointe de sonde dans une solution acide ayant un pH d'environ 1,0 à 4,0 pour éluer l'immunocomplexe à partir de la pointe de sonde ; et
(h) de répétition des étapes (b) à (g) avec un second échantillon liquide dans un second récipient à échantillon dans un second cycle, grâce à quoi l'analyte dans de multiples échantillons liquides est détecté.

2. Procédé selon la revendication 1, dans lequel les courbes d'étalonnage à l'étape (f) sont les mêmes pour tous les cycles de quantification.

3. Procédé selon la revendication 1, dans lequel la solution acide à l'étape (g) a un pH de 1,5 à 2,5.

4. Procédé selon la revendication 1, dans lequel à l'étape (g), la pointe de sonde est exposée à la solution acide une fois pendant 10 secondes à 2 minutes.

5. Procédé selon la revendication 1, dans lequel, à l'étape (g), la pointe de sonde est exposée à un traitement par impulsions de 2 à 5 cycles du traitement par solution acide suivi d'une neutralisation dans le récipient de lecture pendant 10 à 20 secondes.

6. Procédé de détection d'une protéine c-réactive ayant une large gamme de concentrations dans de multiples échantillons liquides, comprenant les étapes :
(i) d'obtention d'une sonde ayant un premier anticorps immobilisé sur la pointe de la sonde, le diamètre de la surface de pointe étant ≤ 5 mm ; le premier anticorps étant un anticorps monoclonal de souris anti-protéine c-réactive humaine CRP30 ;
(ii) de trempage de la sonde dans un récipient de prélecture comprenant une solution aqueuse pour prélire le signal fluorescent de la pointe de sonde,
(iii) de trempage de la pointe de sonde dans un premier récipient à échantillon contenant une première solution d'échantillon ayant un analyte de protéine c-réactive pour lier l'analyte au premier anticorps sur la pointe de sonde ;
(iv) de trempage de la pointe de sonde dans un récipient de réactif contenant une solution de réactif comprenant un second anticorps conjugué avec des marqueurs fluorescents, pour former un complexe immunitaire de l'analyte, du premier anticorps et du second anticorps, le premier anticorps et le second anticorps étant des anticorps contre le analyte ;
(v) de trempage de la pointe de sonde dans un récipient de lavage contenant une solution de lavage pour laver la pointe de sonde ;
(vi) de mesure d'un premier signal fluorescent du premier complexe immunitaire formé sur la pointe de sonde ;
(vii) de trempage de la pointe de sonde dans le même récipient à échantillon pendant une période de temps plus longue que celle de l'étape (iii), et d'écoulement de la solution d'échantillon dans le premier récipient d'échantillon, pour lier l'analyte supplémentaire dans le premier échantillon au premier anticorps sur la pointe de sonde ;
(viii) de répétition de l'étape (iv) avec un temps d'incubation plus long et de répétition de l'étape (v) ;
(ix) de mesure d'un second signal fluorescent du second complexe immunitaire formé sur la pointe de sonde ;
(x) de détermination de la concentration d'analyte dans le premier échantillon en soustrayant d'abord le signal fluorescent prélu de (b) des premier et second signaux fluorescents, puis en quantifiant la concentration d'analyte par rapport à une courbe d'étalonnage d'extrémité supérieure ou une courbe d'étalonnage d'extrémité inférieure ;
(xi) de trempage de la pointe de sonde dans une solution acide ayant un pH d'environ 1,0 à 4,0 pour éluer le complexe immunitaire à partir de la pointe de sonde, et
(xii) de répétition des étapes (ii) à (xi) avec un second échantillon liquide dans un second récipient à échantillon dans un second cycle, grâce à quoi l'analyte dans de multiples échantillons liquides est détecté.

7. Procédé selon la revendication 6, dans lequel les courbes d'étalonnage d'extrémité supérieure et d'extrémité inférieure de l'étape (x) sont les mêmes pour tous les cycles de quantification.

8. Procédé selon la revendication 6, dans lequel les courbes d'étalonnage d'extrémité supérieure et d'extrémité inférieure de l'étape (x) sont des courbes d'étalonnage spécifiques au cycle.

9. Procédé selon la revendication 6, dans lequel la solution acide à l'étape (xi) a un pH de 1,5 à 2,5.

10. Procédé selon la revendication 6, dans lequel, à l'étape (xi), la pointe de sonde est exposée à la solution acide une fois pendant 10 secondes à 2 minutes.

11. Procédé selon la revendication 1 ou 6, dans lequel le premier anticorps est marqué avec de la biotine et est indirectement immobilisé sur la surface de détection revêtue de streptavidine.

12. Cartouche pour un immunodosage de la protéine c-réactive, comprenant (a) un puits de sonde comprenant une sonde et un capuchon, le capuchon étant dans une position fermée pour enfermer la sonde dans le puits de sonde, la sonde ayant une pointe inférieure revêtue d'un premier anticorps ;
le premier anticorps étant un anticorps monoclonal de souris anti-protéine c-réactive humaine CRP30 ;
(b) un puits d'échantillon pour recevoir un échantillon ;
(c) un puits de réactif contenant des réactifs ;
(d) un ou plusieurs puits de lavage contenant chacun une solution de lavage ;
(e) un puits à pH bas comprenant un réactif acide pour fournir un pH de 1 à 4, (f) un puits de neutralisation contenant un réactif tampon ayant un pH de 6,0 à 8,5 ; et
(g) un puits de mesure ayant un fond transmettant la lumière, le puits de mesure contenant une solution aqueuse ;
les ouvertures du puits d'échantillon, du puits de réactif, du puits de mesure et des puits de lavage étant scellées.

13. Cartouche pour un immunodosage de la protéine c-réactive, comprenant (a) un puits de sonde comprenant une sonde et un capuchon, le capuchon étant dans une position fermée pour enfermer la sonde dans le puits de sonde, la sonde ayant une pointe inférieure revêtue d'un premier anticorps, le premier anticorps étant un anticorps monoclonal de souris anti-protéine c-réactive humaine CRP30 ;
(b) un puits d'échantillon pour recevoir un échantillon ;
(c) un puits de réactif contenant des réactifs ;
(d) un ou plusieurs puits de lavage contenant chacun une solution de lavage ;
(e) un puits à pH bas contenant un réactif à pH acide pour fournir un pH de 1 à 4, et (f) un puits de neutralisation et de mesure ayant un fond transmettant la lumière et contenant une solution tampon aqueuse ayant un pH de 6,0 à 8,5 ;
les ouvertures du puits d'échantillon, du puits de réactif, du puits de mesure et des puits de lavage étant scellées.

14. Sonde comprenant un substrat monolithique revêtu d'un anticorps au niveau de la pointe de sonde, dans laquelle la sonde a un rapport longueur/largeur d'au moins 5 à 1, le diamètre de la surface de pointe est ≤ 5 mm, et l'anticorps est un anticorps monoclonal de souris anti-protéine c-réactive humaine CRP30, éventuellement l'anticorps étant marqué avec de la biotine et immobilisé sur la pointe de sonde par de la streptavidine appliquée sur la pointe de sonde.
